# EUROPEAN PATENT SPECIFICATION

(11) **EP 2 178 498 B1**
(45) Date of publication and mention of the grant of the patent: **18.04.2018**
(21) Application number: 08807379.6
(22) Date of filing: 20.08.2008
(51) Int. Cl.: A61Q 5/02, A61Q 5/12, A61K 8/81, A61K 8/02

(54) **METHOD FOR TREATING DAMAGED HAIR**
VERFAHREN ZUR BEHANDLUNG BESCHÄDIGTER HAARE
PROCÉDÉ DE TRAITEMENT DE CHEVEUX ABIMÉS

(30) Priority: 20.08.2007 US 894144; 31.10.2007 US 981036; 31.05.2008 US 131057
(43) Date of publication of application: 28.04.2010
(73) Proprietor: The Procter & Gamble Company, Cincinnati, OH 45202 (US)
(72) Inventor: BROWN, Mark, Anthony, Union, Kentucky 41091 (US); HUTCHINS, Thomas, Allen, Cincinnati, Ohio 45245 (US); PAGE, Steven, Hardy, Lawrenceburg, Indiana 47025 (US); JAIN, Shashi, West Chester, Ohio 45069 (US)
(74) Representative: Briatore, Andrea
(86) International application number: PCT/IB2008/053340
(87) International publication number: WO 2009/024936

(56) References cited:
- WO-A-92/10162
- WO-A-03/039499
- WO-A-03/101410
- US-A- 5 756 436

## Description

### FIELD OF THE INVENTION

The present invention relates to a method for treating damaged hair, particularly chemically treated hair, by applying a shampoo composition which contains lyotropic liquid crystals to the hair, as well as to articles of manufacture comprising the shampoo composition, and methods of promoting the sale thereof.

### BACKGROUND OF THE INVENTION

Hair can suffer damage from a number of sources, such as environmental exposure to ultraviolet radiation and chlorine, chemical treatment, i.e., bleaching, coloring, perming, as well as mechanical influences, i.e., prolonged use of heated styling appliances. Damage due to chemical treatment is quite common. For example, over 70% of women in the developed world have colored their hair at least once, and 30% of women color their hair every 6 weeks.

Hair coloring, particularly permanent coloring or bleaching, i.e., oxidative dyeing, leads to irreversible physico-chemical changes in the hair. Typically, during the permanent coloring or bleaching processes, at least two components are mixed together prior to application to the hair. These components usually include one component containing an oxidizing agent, such as hydrogen peroxide, and a second component containing an alkalizer buffered at a high pH, typically around 8.5 to 10.5, and, optionally, dyeing materials, such as oxidative dye precursors and couplers. The mixture is left for a period of time suitable to allow the required color transformation to occur. After the coloring or bleaching process, the surface energy of the hair increases; the hair becomes more hydrophilic, as compared to non-colored hair. The change in hair hydrophilicity is believed to be due to, among other things, the oxidation of the keratin-keratin cystine amino acids within the hair, creating more hydrophilic cysteic acid amino acid residues, and to the removal by hydrolysis of the hydrophobic F-Layer of the hair. As a result, the hair becomes increasingly hydrophilic, feels drier and rougher, and is more susceptible to further damage, including breakage and frayed or split ends.

It is known to use hair conditioners to treat damaged hair, including chemically treated hair. More specifically, post-shampoo application of hair conditioners, such as leave-on or rinse-off products, is known and hair conditioning shampoos, which cleanse and condition the hair, are also known. Polydimethylsiloxanes (PDMS), which are highly hydrophobic, are often employed as conditioning agents in such products to improve hair feel by making hair more hydrophobic. Hydrophobic PDMS increase the hydrophobicity of hair, thereby providing a smooth, soft feel to the hair and reducing combing forces for wet and dry hair. It is known, however, that, in the case of more hydrophilic, damaged hair, such as that obtained after one or more oxidative colorings, PDMS deposition is greatly reduced and cannot provide the same benefit in hair condition as for undamaged or virgin hair.

US 5756436 A1 discloses aqueous conditioning shampoo compositions which comprise an anionic detersive surfactant component; dispersed, liquid, droplets of a water insoluble, hair conditioning agent having a number average particle diameter of from about 0.01 microns to about 2000 microns; from about 0.025 to about 5 wt.-% of an organic, cationic, non crosslinked, deposition or conditioning polymer having a cationic charge density of from about 4 meq/gm to about 7 meq/gm and an average molecular weight of from about 1,000 to about 1 million.

WO 92/10162 A discloses hair conditioning shampoo compositions comprising: (a) from about 5 to about 50 wt.-% of an anionic surfactant component; (b) from about 0.1 to about 10 wt.-% of a dispersed, insoluble, nonvolatile, nonionic silicone hair conditioning agent; (c) from about 0.05 to about 10 wt.-% of soluble, organic, polymeric cationic hair conditioning agent, said polymeric, cationic hair conditioning agent consisting essentially of one or more cationic, hair conditioning polymers, said cationic hair conditioning polymers having quaternary ammonium or cationic amino moieties, or a mixture thereof, an open chain backbone, and a cationic charge density of about 3.0 meq/gram or less; and (d) an aqueous carrier.

Based on the foregoing, there is a need for a method for treating damaged hair, particularly chemically treated hair, to reduce its surface energy, thereby increasing its hydrophobicity and restoring its natural smooth, lubricious feel. Furthermore, there is a need for a method of communicating to a consumer the ability of a shampoo composition to treat damaged hair, particularly chemically treated hair, and restore its natural smooth, lubricious feel.

### SUMMARY OF THE INVENTION

The present invention relates to the use of a rinse-off shampoo composition for reducing, upon treatment, the surface energy of damaged hair being chemically treated hair, as compared to that measured prior to the treatment, wherein the composition comprises:
i from 5% to 50% by weight of an anionic surfactant;
ii from 0.025% to 5% by weight of a synthetic cationic polymer having a cationic charge density of from 4 to 7 meq/gm, wherein said synthetic cationic polymer forms lyotropic liquid crystals upon combination with said anionic surfactant; and
iii water;

The present disclosure also relates to an article of manufacture useful for treating damaged hair, where the damaged hair is chemically treated hair, comprising (a) a package; (b) said package containing a shampoo composition, wherein said shampoo composition comprises lyotropic liquid crystals; and (c) information to communicate to consumers the ability of the shampoo composition to treat chemically treated hair.

The present disclosure also relates to methods of promoting the sale of shampoo compositions which are useful for treating damaged hair, where the damaged hair is chemically treated hair. The methods include a variety of steps to inform a consumer of the ability of the present shampoo compositions and articles to treat chemically treated hair and encourage the consumer to use them to treat chemically treated hair.

### DETAILED DESCRIPTION OF THE INVENTION

It has been discovered that compositions which form lyotropic liquid crystals are particularly useful in treating damaged hair, where the damage is due to a chemical treatment.

A liquid crystalline state exists structurally between the solid crystalline phase and the liquid phase (i.e. an intermediate between the three dimensionally ordered crystalline state and the completely disordered liquid state).

The term "liquid crystal", as used herein, means a material having phases that are ordered and/or crystalline in only one or two of their three possible orthogonal directions and are disordered (random and/or liquid-like) in the other dimensions.

The term "lyotropic", as used herein, means that the ordering effects of a material are induced by changing both its concentration and temperature.

The term "nonvolatile" refers to any material having little or no significant vapor pressure under ambient conditions, and a boiling point under one atmosphere (atm) preferably at least about 250°C. The vapor pressure under such conditions is preferably less than about 0.2 mm.

The term "polymer", as used herein, shall include materials whether made by polymerization of one type of monomer or made by two (*i.e*., copolymers) or more types of monomers.

The term "water soluble", as used herein, means that the polymer is soluble in water in the present composition. In general, the polymer should be soluble at 25°C at a concentration of 0.1% by weight of the water solvent, preferably at 1%, more preferably at 5%, more preferably at 15%.

The term "chemically treated hair", as used herein, shall include color-treated hair, bleached hair, highlighted hair, permed hair, or hair that has been treated with any combination of such chemical treatments, i.e., permed and color-treated hair.

All percentages, parts and ratios are based upon the total weight of the compositions of the present invention, unless otherwise specified. All such weights as they pertain to listed ingredients are based on the active level and therefore do not include solvents or by-products that may be included in commercially available materials, unless otherwise specified.

All molecular weights as used herein are weight average molecular weights expressed as grams/mole, unless otherwise specified.

### Shampoo Composition

As discussed hereinbefore, the present invention includes a shampoo composition which contains lyotropic liquid crystals. The liquid crystals may form upon combination of the detersive surfactant component and cationic polymer discussed hereinafter. It has been discovered that application of the shampoo composition described herein to damaged hair, particularly chemical treated hair, reduces the surface energy of the hair, thereby increasing its hydrophobicity and restoring its natural smooth, lubricious feel. Typically, the application of the shampoo composition to damaged hair, particularly chemical treated hair, reduces the surface energy of the hair by at least about 10%, more commonly by at least about 50%, preferably by at least about 100% and up to about 5000%.

### Anionic Surfactant Component

The shampoo compositions of the invention comprise an anionic detersive surfactant component to provide cleaning performance to the composition and to aid in formation of the lyotropic liquid crystalline phase. The anionic surfactant component comprises an anionic detersive surfactant, and optionally, a zwitterionic and/or amphoteric detersive surfactant, which has an attached group that is anionic at the pH of the composition. Such surfactants should be physically and chemically compatible with the essential components described herein or should not otherwise unduly impair product stability, aesthetics, or performance.

Suitable anionic detersive surfactant components include those which are known for use in hair care or other shampoo cleansing compositions. The concentration of the anionic surfactant component generally ranges from about 5% to about 50%, preferably from about 8% to about 30%, more preferably from about 10% to about 25%, even more preferably from about 12% to about 20%, by weight of the composition.

Preferred anionic detersive surfactants for use in the shampoo compositions include ammonium lauryl sulfate, ammonium laureth sulfate, triethylamine lauryl sulfate, triethylamine laureth sulfate, triethanolamine lauryl sulfate, triethanolamine laureth sulfate, monoethanolamine lauryl sulfate, monoethanolamine laureth sulfate, diethanolamine lauryl sulfate, diethanolamine laureth sulfate, lauric monoglyceride sodium sulfate, sodium lauryl sulfate, sodium laureth sulfate, potassium lauryl sulfate, potassium laureth sulfate, sodium lauryl sarcosinate, sodium lauroyl sarcosinate, lauryl sarcosine, cocoyl sarcosine, ammonium cocoyl sulfate, ammonium lauroyl sulfate, sodium cocoyl sulfate, sodium lauroyl sulfate, potassium cocoyl sulfate, potassium lauryl sulfate, triethanolamine lauryl sulfate, triethanolamine lauryl sulfate, monoethanolamine cocoyl sulfate, monoethanolamine lauryl sulfate, sodium tridecyl benzene sulfonate, sodium dodecyl benzene sulfonate, and combinations thereof.

Suitable amphoteric or zwitterionic detersive surfactants for use in the shampoo composition herein include those which are known for use in hair care or other personal care cleansing compositions and those which contain a group that is anionic at the pH of the shampoo composition. The concentration of such amphoteric detersive surfactants preferably ranges from about 0.5 % to about 20%, preferably from about 1% to about 10% by weight of the composition. Non-limiting examples of suitable zwitterionic or amphoteric surfactants are described in U.S. Patent Nos. 5,104,646 and 5,106,609.

The shampoo compositions may further comprise additional surfactants for use in combination with the anionic detersive surfactant component described hereinbefore. Suitable optional surfactants include nonionic surfactants, cationic surfactants, and combinations thereof. Any such surfactant known in the art for use in hair or personal care products may be used, provided that the optional additional surfactant is also chemically and physically compatible with the essential components of the shampoo composition or does not otherwise unduly impair product performance, aesthetics or stability. The concentration of optional additional surfactants in the shampoo composition may vary with the cleansing or lather performance desired, the optional surfactant selected, the desired product concentration, the presence of other components in the composition, and other factors well known in the art.

Non limiting examples of other anionic, zwitterionic, amphoteric, or optional additional surfactants suitable for use in the shampoo compositions are described in U.S. Patent Nos. 3,929,678; 2,658,072; 2,438,091; and 2,528,378.

### Synthetic Cationic Polymer

The cationic polymer described herein aids in providing damaged hair, particularly chemically treated hair, with a surrogate hydrophobic F-layer. The microscopically thin F-layer provides natural weatherproofing, while helping to seal in moisture and prevent further damage. Chemical treatments damage the hair cuticle and strip away its protective F-layer. As the F-layer is stripped away, the hair becomes increasingly hydrophilic. It has been found that when lyotropic liquid crystals are applied to chemically treated hair, the hair becomes more hydrophobic and more virgin-like, in both look and feel. Without being limited to any theory, it is believed that the lyotropic liquid crystal complex creates a hydrophobic layer or film, which coats the hair fibers and protects the hair, much like the natural F-layer protects the hair. The hydrophobic layer returns the hair to a generally virgin-like, healthier state.

Lyotropic liquid crystals are formed by combining the synthetic cationic polymers described herein with the aforementioned anionic detersive surfactant component of the shampoo composition. The synthetic cationic polymer has a relatively high charge density. It should be noted that some synthetic polymers having a relatively high cationic charge density do not form lyotropic liquid crystals, primarily due to their abnormal linear charge densities. Such synthetic cationic polymers are described in WO 94/06403 to Reich et al. The synthetic polymers described herein can be formulated in a stable shampoo composition that provides improved conditioning performance, with respect to damaged hair. In some embodiments, the synthetic cationic polymer may be formed from
i) one or more cationic monomer units, and optionally
ii) one or more momomer units bearing a negative charge, and/or
iii) a nonionic momomer,
wherein the subsequent charge of the copolymer is positive. The ratio of the three types of monomers is given by "m", "p" and "q" where "m" is the number of cationic monomers, "p" is the number of momomers bearing a negative charge and "q" is the number of nonionic momomers.

The concentration of the cationic polymers ranges about 0.025% to about 5%, preferably from about 0.1% to about 3%, more preferably from about 0.2% to about 1%, by weight of the shampoo composition.

The cationic polymers have a cationic charge density of from 4 meq/gm to 7 meq/gm. In some embodiments, the cationic charge density is about 6.2 meq/gm. The polymers also have a molecular weight of from about 1,000 to about 5,000,000, more preferably from about 10,000 to about 2,000,000, most preferably 100,000 to about 2,000,000.

In one embodiment, the cationic polymers are water soluble or dispersible, non-crosslinked, synthetic cationic polymers having the following structure: 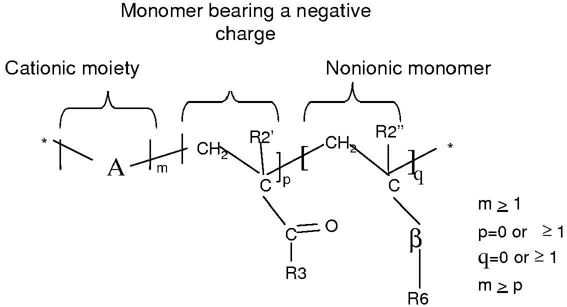
Where A, may be one or more of the following cationic moieties: 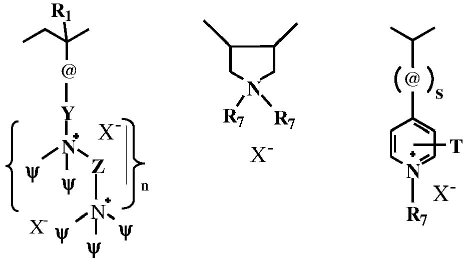
Where @ = amido, alkylamido, ester, ether, alkyl or alkylaryl.
Where Y = C1-C22 alkyl, alkoxy, alkylidene, alkyl or aryloxy.
Where ψ = C1-C22 alkyl, alkyloxy, alkyl aryl or alkyl aryloxy.
Where Z = C1-C22 alkyl, alkyloxy, aryl or aryloxy.
Where R1 = H, C1-C4 linear or branched alkyl.
Where s = 0 or 1, n = 0 or ≥ 1.
Where T and R7 = C1-C22 alkyl.
Where X- = halogen, hydroxide, alkoxide, sulfate or alkylsulfate.

Where the monomer bearing a negative charge is defined by R2' = H, C1-C4 linear or branched alkyl and R3 as: 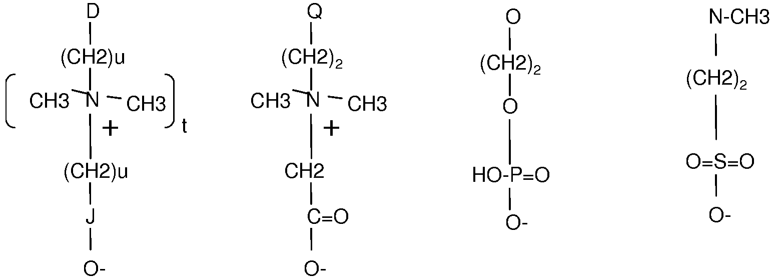
Where D = O, N, or S.
Where Q = NH₂ or O.
Where u = 1-6.
Where t = 0-1.
Where J = oxygenated functional group containing the following elements P, S, C.

Where the nonionic monomer is defined by R2" = H, C1-C4 linear or branched alkyl, R6 = linear or branched alkyl, alkyl aryl, aryl oxy, alkyloxy, alkylaryl oxy and β is defined as 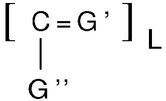 Where G' and G" are, independently of one another, O, S or N-H and L =0 or 1.

Examples of cationic monomers include aminoalkyl (meth)acrylates, (meth)aminoalkyl (meth)acrylamides; monomers comprising at least one secondary, tertiary or quaternary amine function, or a heterocyclic group containing a nitrogen atom, vinylamine or ethylenimine; diallyldialkyl ammonium salts; their mixtures, their salts, and macromonomers deriving from therefrom.

Further examples of cationic monomers include dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide, ethylenimine, vinylamine, 2-vinylpyridine, 4- vinylpyridine, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride, diallyldimethyl ammonium chloride.

Preferred cationic monomers comprise a quaternary ammonium group of formula -NR₃⁺, wherein R, which is identical or different, represents a hydrogen atom, an alkyl group comprising 1 to 10 carbon atoms, or a benzyl group, optionally carrying a hydroxyl group, and comprise an anion (counter-ion). Examples of anions are halides such as chlorides, bromides, sulphates, hydrosulphates, alkylsulphates (for example comprising 1 to 6 carbon atoms), phosphates, citrates, formates, and acetates.

Preferred cationic monomers include trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride.

More preferred cationic monomers include trimethyl ammonium propyl (meth)acrylamido chloride.

Examples of monomers bearing a negative charge include alpha ethylenically unsaturated monomers comprising a phosphate or phosphonate group, alpha ethylenically unsaturated monocarboxylic acids, monoalkylesters of alpha ethylenically unsaturated dicarboxylic acids, monoalkylamides of alpha ethylenically unsaturated dicarboxylic acids, alpha ethylenically unsaturated compounds comprising a sulphonic acid group, and salts of alpha ethylenically unsaturated compounds comprising a sulphonic acid group.

Preferred monomers with a negative charge include acrylic acid, methacrylic acid, vinyl sulphonic acid, salts of vinyl sulfonic acid, vinylbenzene sulphonic acid, salts of vinylbenzene sulphonic acid, alpha-acrylamidomethylpropanesulphonic acid, salts of alpha-acrylamidomethylpropanesulphonic acid, 2-sulphoethyl methacrylate, salts of 2-sulphoethyl methacrylate, acrylamido-2-methylpropanesulphonic acid (AMPS), salts of acrylamido-2-methylpropanesulphonic acid, and styrenesulphonate (SS).

Examples of nonionic monomers include vinyl acetate, amides of alpha ethylenically unsaturated carboxylic acids, esters of an alpha ethylenically unsaturated monocarboxylic acids with an hydrogenated or fluorinated alcohol, polyethylene oxide (meth)acrylate (i.e. polyethoxylated (meth)acrylic acid), monoalkylesters of alpha ethylenically unsaturated dicarboxylic acids, monoalkylamides of alpha ethylenically unsaturated dicarboxylic acids, vinyl nitriles, vinylamine amides, vinyl alcohol, vinyl pyrolidone, and vinyl aromatic compounds.

Preferred nonionic monomers include styrene, acrylamide, methacrylamide, acrylonitrile, methylacrylate, ethylacrylate, n-propylacrylate, n-butylacrylate, methylmethacrylate, ethylmethacrylate, n-propylmethacrylate, n-butylmethacrylate, 2-ethyl-hexyl acrylate, 2-ethyl-hexyl methacrylate, 2-hydroxyethylacrylate and 2-hydroxyethylmethacrylate.

The anionic counterion (X-) in association with the synthetic cationic polymers may be any known counterion so long as the polymers remain soluble or dispersible in water, in the shampoo composition, or in a coacervate phase of the shampoo composition, and so long as the counterions are physically and chemically compatible with the essential components of the shampoo composition or do not otherwise unduly impair product performance, stability or aesthetics. Non limiting examples of such counterions include halides (e.g., chlorine, fluorine, bromine, iodine), sulfate and methylsulfate.

### Optional Ingredients

The shampoo composition may further comprise optional ingredients selected from the group consisting of oily conditioning agents, hydrocarbon oils, polyolefins, fatty esters, fluorinated conditioning compounds, fatty alcohols, quaternary ammonium compounds, polyethylene glycols, anti-dandruff actives, anti-microbial actives, inorganic or synthetic particles, opacifying agents, suspending agents, propellants, paraffinic hydrocarbons, mono or divalent salts, fragrances, vitamins, chelating agents, colorants, pigments, dyes, phase separation initiators such as electrolytes, and mixtures thereof. These optional components are described in detail in U.S. Patent Publication No. 2003/0223951A1. Suspending agents are described in U.S. Patent No. 5756436. Such optional ingredients may be present in an amount of from about 0.1% to about 5% by weight of the shampoo composition.

### Silicone Conditioning Agent

If an oily conditioning agent is included, it is preferably in the form of a water-insoluble silicone conditioning agent. The silicone conditioning agent may comprise volatile silicone, non-volatile silicone, or combinations thereof. Non-volatile silicone conditioning agents are preferred. If volatile silicones are present, their presence is typically incidental to their use as a solvent or carrier for commercially available forms of non-volatile silicone materials, such as silicone gums and resins. The silicone conditioning agent particle may be in the form of a silicone resin, or it may be in the form of a silicone fluid (ie. dimethicone droplets).

Non-limiting examples of suitable silicone conditioning agents and optional suspending agents for the silicone are described in U.S. Reissue Patent No. 34,584, U.S. Patent Nos. 5,104,646, and 5,106,609. The silicone conditioning agents for use in the compositions preferably have a viscosity, as measured at 25°C, of from about 20 to about 2,000,000 centistokes ("csk"), more preferably from about 1,000 to about 1,800,000 csk, even more preferably from about 5,000 to about 1,500,000 csk, more preferably from about 10,000 to about 1,000,000 csk.

The oil droplets preferably have a volume average particle diameter of from about 0.01 microns to about 100 microns. For small particle application to hair, the volume average particle diameters preferably range from about 0.01 microns to about 4 microns, more preferably from about 0.01 to about 2 microns, even more preferably from about 0.01 microns to about 0.5 microns. For larger particle application to hair, the volume average particle diameters preferably range from about 4 microns to about 50 microns, more preferably from about 9 microns to about 45 microns, even more preferably from about 25 microns to about 40 microns, still more preferably from about 25 microns to about 35 microns. The more preferred range of about 25 microns to about 35 microns maximizes silicone deposition efficiency and shampoo phase stability. Particle size is measured using the LA-910 Particle Size Analyzer, manufactured by Horiba.

Non-volatile silicone oils suitable for use in the compositions may be selected from organo-modified silicones and fluoro-modified silicones. In one embodiment, the non-volatile silicone oil is an organo-modified silicone which comprises an organo group selected from the group consisting of alkyl groups, alkenyl groups, hydroxyl groups, amine groups, quaternary groups, carboxyl groups, fatty acid groups, ether groups, ester groups, mercapto groups, sulfate groups, sulfonate groups, phosphate groups, propylene oxide groups, and ethylene oxide groups.

In a preferred embodiment, the non-volatile silicone oil is polydimethylsiloxane.

Silicone fluids suitable for use in the compositions are disclosed in U.S. Patent Nos. 2,826,551; 3,964,500; and 4,364,837, British Patent No 849,433, and *Silicon Compounds,* Petrarch Systems, Inc. (1984).

### Method for Treating Damaged Hair

The compositions described herein are particularly useful in treating damaged hair, particularly chemically treated hair. As discussed above, such hair is increasingly hydrophilic (increased surface energy), as compared to virgin hair.

The method of treating damaged hair, particularly chemically treated hair, comprises the steps of contacting the hair, which has preferably been wetted with water, with an effective amount of the shampoo composition described herein. After contacting the hair with the shampoo composition, the composition is rinsed from the hair. Effective amounts of the shampoo composition generally range from about 1 gm to about 50 gm, preferably from about 1 gm to about 20 gm. Application to the hair typically includes working the composition through the hair such that most or all of the hair is contacted with the composition.

### Article of Manufacture for Treating Damaged Hair

The present disclosure also relates to an article of manufacture useful for treating damaged hair, particularly chemically treated hair. The article comprises a package which contains the shampoo composition described herein. The package is in association with information or instructions, in the form of indicia, which informs the consumer that the shampoo composition will treat and improve the quality of damaged hair, particularly chemically treated hair, i.e., by providing a more lubricious feel and increased shine to the hair. The indicia may be in the form of words, pictures, symbols, or the like. Furthermore, the package may include a claim of superiority over other shampoo compositions. As used herein, the phrase "in association with" means the information or instructions are either directly printed on the package itself or presented in a different manner, including, but not limited to, as promotional material to communicate the information or instructions to a consumer. The information or instructions are important to encourage consumers, especially those with chemically treated hair, to use the shampoo composition described herein.

In another embodiment, the package may bear information that informs the consumer that the shampoo composition provides one or more benefit selected from providing hair with a protective hydrophobic layer, restoring hair to a virgin-like state, restoring hair shine, or combinations thereof. The lyotropic liquid crystals present in the shampoo compositions herein have been found to provide these and other benefits to damaged hair, particularly chemically treated hair.

The package should be any package suitable for containing liquid compositions. In the case of shampoo compositions, such packages are typically formed from petroleum-based plastics such as PET.

### Method of Promoting the Sale of Article of Manufacture for Treating Damaged Hair

The present disclosure also relates to methods for promoting the sale of the aforementioned articles of manufacture. The present methods generally comprise providing promotional materials to consumers by a variety of steps to inform them of the benefits of the present shampoo compositions for damaged hair, especially chemically treated hair, and particularly to communicate the function of lyotropic liquid crystals in treating damaged hair.

In one embodiment, the method comprises promoting the sale of a shampoo product which contains lyotropic liquid crystals, comprising the steps of (a) displaying, shelving, or merchandising the shampoo product in a retail store; and/or (b) providing promotional materials to consumers, wherein said promotional materials comprise information regarding the shampoo product's ability to treat chemically treated hair and/or an instruction to apply the shampoo product to hair which has been chemically treated.

In another embodiment, the method includes sending promotional materials directly to consumers via mail or electronic mail. The promotional materials can also include samples of the shampoo compositions herein, or articles, and can include discount coupons, which the consumer can redeem upon purchasing the present shampoo compositions or articles.

In yet another embodiment, the method includes providing promotional materials to a hair styling salon, which is intended to encourage the stylist to provide the promotional materials or information to his or her customers, preferably to customers with damaged hair. For example, a consumer with chemically treated hair may have the chemical treatment performed by a professional stylist. It is believed that such stylists would be enabled to effectively communicate the benefits of lyotropic liquid crystals to customers upon receipt and review of the promotional materials of the present invention.

### Method of Manufacture

The compositions, in general, may be made by mixing together at elevated temperature, e.g., about 72°C, water and surfactants along with any solids (e.g. amphiphiles) that need to be melted, to speed mixing into the personal cleansing composition. The ingredients are mixed thoroughly at the elevated temperature and then cooled to ambient temperature. Additional ingredients, including electrolytes, polymers, and particles, may be added to the cooled product. The silicone may be emulsified at room temperature in concentrated surfactant and then added to the cooled product.

All exemplified amounts are listed as weight percents and exclude minor materials such as diluents, preservatives, color solutions, imagery or conceptual ingredients, botanicals, and so forth, unless otherwise specified.

### Testing Methods

The following procedures were used to evaluate the compositions of the invention. Specifically, surface energy is measured according to the method described below. A reduction in surface energy corresponds to an increase in hydrophobicity. Relative to the data below, the evaluated hair samples ("switches") are prepared or obtained according to the following techniques.

### Preparation of Damaged Hair Switches:

Virgin (commonly referred to as special quality hair or special quality virgin hair) hair, moderately damaged hair, and bleached hair (commonly referred to as low lift substrate hair) are purchased from International Hair Importers & Products Inc., 87-29 Myrtle Ave., Glendale, NY 11385, under the codes SPQ, for the virgin hair, SPQLLS, for the bleached hair, and PGMDST, for the moderately damaged hair.

Permed hair is prepared using commercially available Option 1™ Perm (Innovative Styling Options, Inc., Darien, CT). 4-gram, 8-inch switches of virgin hair are first rinsed, with water, for about 30 seconds. After rinsing, the switches are sandwiched between one's index and middle fingers and pulled through the fingers to remove excess water. Each switch is then blotted gently with a paper towel to dry the switch. The switches are then placed on a sheet of plastic wrap on a tray and a syringe is used to apply .10 cubic centimeters (cc) of Option 1™ Prewrap to each switch. The Prewrap is then worked into each hair switch. Then, 2.0 cc of Option 1™ Waving Lotion is applied to each switch and gently worked through each switch. The treated switches are then left to rest at room temperature for about 20 minutes. Afterwards, the switches are rinsed with water, for about 30 seconds. After rinsing, the switches are sandwiched between one's index and middle fingers and pulled through the fingers to remove excess water. Each switch is then blotted gently with a paper towel to dry the switch. The switches are then placed on a clean sheet of plastic wrap on a tray. Next, 2.0 cc of Option 1™ Neutralizer solution is applied with a syringe to each switch. The treated switches are then left to rest at room temperature for about 5 minutes. Afterwards, the switches are rinsed with water, for about 30 seconds. Excess water is removed from the rinsed switches and the switches are hung to dry. Water used for rinsing hair switches is typically at a temperature of about 100°F and a pressure of about 1.5 gal/min.

Color-treated hair is prepared using commercially available Nice n' Easy® hair color. 4-gram, 8-inch switches of bleached hair (purchased from International Hair Importers & Products Inc., 87-29 Myrtle Ave., Glendale, NY 11385) are placed on a sheet of plastic wrap on a tray. The hair colorant is prepared according to the instructions provided with the colorant. 12 cc of colorant are applied with a syringe to the front side of each switch on the tray. The colorant is then massaged into each switch, for about 30 seconds per switch, making sure that the hair fibers are separated and that the colorant is applied evenly to each strand of hair. The switches are then turned over and 12 cc of colorant are applied to the back side of each switch. Again, the colorant is massaged or worked into the hair, for about 30 seconds per switch, i.e., by spreading the switch back and forth, making sure that the hair fibers are separated and that the colorant is applied evenly to each strand of hair. The treated switches are then wrapped in plastic wrap and placed in an oven, at about 30°C to about 32°C, for about 30 minutes. Afterwards, the front sides of the switches are rinsed in water for about 1 minute, while running one's fingers through the hair. After rinsing, the switches are sandwiched between one's index and middle fingers and pulled through the fingers to remove excess water. The switches are then turned over and the back sides of the switches are rinsed in water for about 1 minute, while running one's fingers through the hair. After this rinse, the switches are sandwiched between one's index and middle fingers and pulled through the fingers to remove excess water. Water used for rinsing hair switches it typically at a temperature of about 100°F and a pressure of about 1.5 gal/min.

The technique for treating the switches with a shampoo composition is discussed below.

### Application of Shampoo Compositions to Hair Samples:

Hair switches are hung over a sink and pre-wetted with water for about 30 seconds. The switches are then sandwiched between one's index and middle fingers and pulled through the fingers to remove excess water. 0.4 cc of shampoo composition is applied to the front side of each hair switch, in a zig-zag manner down the length of each switch. The shampoo is brushed into each hair switch, for about 30 seconds, using a small, Goody®, stiff-bristle, plastic brush. Each hair switch is then rinsed with water for about 30 seconds. The switches are then sandwiched between one's index and middle fingers and pulled through the fingers to remove excess water. The hair switches are then turned over and 0.4 cc of shampoo composition is applied to the back side of each hair switch, in a zig-zag manner down the length of each switch. Each hair switch is then rinsed with water for about 30 seconds. The switches are then sandwiched between one's index and middle fingers and pulled through the fingers to remove excess water. The hair switches are then air-dried. Water used for pre-wetting and rinsing hair switches is typically at a temperature of about 100°F and a pressure of about 1.5 gal/min. The water is typically at a hardness of about 7 grains/gallon to about 13 grains/gallon.

The above-steps, with the exception of the pre-wetting step, are repeated four times for each hair switch. After the fourth repetition, the surface energy of each hair switch is measured, according to the protocol that follows.

In the data that follows, the hair switches are each treated with a different shampoo composition, represented in the tabulated data. One switch is treated with the shampoo composition of Example #15 below. Another hair switch is treated with the shampoo composition of Example #5 below. A third hair switch is treated with the shampoo composition of Example #11 below. A fourth hair switch is treated with the shampoo composition of Example #20 below. A final hair switch is treated with the shampoo of Comparative Example #21. Treatment of the switches with shampoo composition involves the steps described below.

### Measurement of Surface Energy of Hair:

Two hair strands are suspended, parallel to each other and the ground, 18.0 ± 0.5 µm apart from each other, i.e., in accordance with the following illustration: 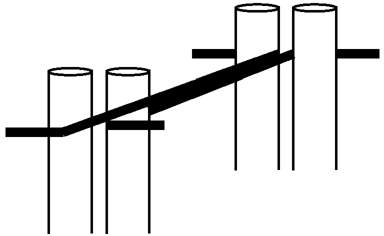 0.2 ± 0.01 µL of solvent is dangled above the hair from the tip of a stainless steel blunt tipped 27 gauge needle. The drop is carefully lowered until it gently makes contact with the 2 hairs. The needle is then slowly retracted leaving the drop on the hairs. Video imaging of the drop making contact with hairs, detaching from the needle, and sitting on the hair up to 15 s after detaching from the needle at 10 images per second is recorded, using a FTA200 Dynamic Contact Angle Analyzer (First Ten Angstroms, Porstmouth, VA). Imaging is performed looking across the length of the parallel hairs with zero look down angle on the drop. The contact angle of the solvents on the hair is determined from the image immediately after the drop has ceased vibrating due to detachment from the needle. Contact angles are determined using commercially available software (First Ten Angstroms, Software version 2.0, Build number 303) in the nonspherical mode for drop shape analysis. Solvents used were 99+% hexadecane and ultrapure water. Fowkes equation of state was used to convert contact angles into apparent surface energies (F.M. Fowkes. Ind. Eng. Chem. 56 (1964) 40).

The tabulated readings below represent the average of 10 total readings, taken on 10 pairs of hair strands, the strands derived from hair switches prepared and treated according to the techniques described above.

**Surface Energy**

| Chemically Treated Hair | Surface Energy of Hair (mJ/m²) Before Treatment* | Surface Energy of Hair (mJ/m²) After Treatment with Example #5 | Surface Energy of Hair (mJ/m²) After Treatment with Comparative Example #21 | Surface Energy of Hair (mJ/m²) After Treatment with Example #15 | Surface Energy of Hair (mJ/m²) After Treatment with Example #11 | Surface Energy of Hair (mJ/m²) After Treatment with Comparative Example #20 |
|---|---|---|---|---|---|---|
| Permed | 0.62 | 0.02 | 0.07 | 0.03 | 0.04 | 0.01 |
| Bleached | 1.2 | 0.20 | 11.63 | 0.32 | 0.05 | 12.31 |
| Colored-Treated | 1.8 | 0.20 | 2.97 | 1.60 | 0.06 | 2.20 |

| | | | | | | |
|---|---|---|---|---|---|---|
| *Permed and color-treated hair is washed six times with a clarifying shampoo, such as Pantene® Purity Clarifying Shampoo, to remove residual perming solution or dye on the surface of the hair, prior to taking a surface energy measurement. | | | | | | |

| | **Example #** |
|---|---|
| (30:70) AM:MAPTAC (4.0 meq/g, 227 meq/Å) | 15 |
| DADMAC (6.2 meq/g, 162 meq/Å) | 5 |
| (10:90) BEM:MAPTAC (2.6 meq/g, 292 meq/Å) | 11 |
| (50:50) AM:MAPTAC (3.4 meq/g, 162 meq/Å) | 20 |
| CATIONIC-POLYMER-FREE | 21 |

The following examples are representative of suitable shampoo compositions for use in the method of treating damaged hair according to the present invention. Also included are comparative examples of non-representative shampoo compositions.

### Non-limiting Examples

**Hompolymers**

| EXAMPLE COMPOSITION | **1** | **2** | **3** | **4** | **5** | **6** | **7** | **8** | **9** | **10** |
|---|---|---|---|---|---|---|---|---|---|---|
| Ammonium Laureth Sulfate (AE₃S) | 6.50 | 6.00 | | | 6.00 | 7.50 | 7.50 | | | |
| Ammonium Lauryl Sulfate (ALS) | 8.10 | 10.00 | | | 10.00 | 6.50 | 6.50 | | | |
| Sodium Laureth Sulfate (SE₃S) | | | 6.00 | 6.50 | | | | 6.00 | 6.00 | 6.50 |
| Sodium Lauryl Sulfate (SLS) | 1.40 | | 10.00 | 5.50 | | | | 7.00 | 10.00 | 5.50 |
| Sodium Lauroamphoacetate ⁽¹⁴⁾ | | | | | | | | | | 2.00 |
| Cocaminopropionic Acid ⁽¹⁵⁾ | | | | 1.00 | | | | | | |
| Cocamidopropyl Betaine ⁽¹⁶⁾ | | | | 1.00 | | | | 2.00 | | |
| Cocamide MEA | 1.00 | | | 0.80 | | 0.80 | 0.80 | 0.85 | | 0.80 |
| Cetyl Alcohol | 0.35 | | | 0.60 | | 0.60 | 0.60 | | | 0.60 |
| Lauryl Alcohol | 0.20 | | | 0.35 | | | | | | 0.35 |
| Laureth-4 Alcohol | | 0.90 | 0.90 | | 0.90 | | | | 0.90 | |
| Dihydrogenated Tallowamidoethyl Hydroxyethylmonium Methosulfate ⁽¹⁷⁾ | | | | 0.15 | | 0.15 | 0.15 | | | 0.15 |
| 1-Propanaminium, N,N,N-trimethyl-3-[(2-methyl-1-oxo-2-propenyl)amino]-, chloride; (Poly(Methacrylamidopropyl trimethyl ammonium chloride)) ^{(1,2)} | 0.40 ⁽¹⁾ | | | | | | | | | |
| Methacryloamidopropyl-pentamethyl-1,3-propylene-2-ol-ammonium dichloride ⁽³⁾ | | | | | | 0.40 | | | | |
| N,N,N,N',N',N",N"-heptamethyl-N"-3-(1-oxo-2-methyl-2-propenyl)aminopropyl-9-oxo-8-azo-decane-1,4,10-triammonium trichloride ⁽¹⁸⁾ | | | | | | | 0.40 | | | |
| diallyldimethyl ammonium chloride ^{(4, 5)} | | 0.10 ⁽⁵⁾ | 0.25 ⁽⁵⁾ | 0.50 ⁽⁴⁾ | 0.25 ⁽⁵⁾ | | | 0.10 ⁽⁵⁾ | 0.25 ⁽⁵⁾ | |
| [(2-methacryloyloxy)ethyl]trimethylammo nium methylsulfate homopolymer ⁽⁶⁾ | | | | | | | | | | 0.05 |
| Ethylene Glycol Distearate | | | 1.50 | 1.50 | | 1.50 | 1.50 | 1.50 | 1.50 | 1.50 |
| Trihydroxystearin ⁽⁷⁾ | 0.25 | 0.10 | | | 0.10 | | | | | |
| Polyethylene Glycol (14000) ⁽⁸⁾ | | | | 0.17 | | 0.17 | 0.17 | | | 0.17 |
| Fragrance | 0.55 | 0.60 | 0.65 | 0.55 | 0.60 | 0.55 | 0.55 | 0.65 | 0.65 | 0.55 |
| Sodium Chloride | 0.30 | 0.40 | 1.40 | 0.80 | 0.40 | 0.80 | 0.80 | 1.40 | 1.40 | 0.80 |
| Ammonium Xylenesulfonate | 0.20 | | | | | | | | | |
| Citric Acid | 0.04 | 0.04 | 0.22 | 0.22 | 0.04 | 0.04 | 0.04 | 0.22 | 0.22 | 0.22 |
| Sodium Citrate | 0.40 | 0.40 | | | 0.40 | 0.40 | 0.40 | | | |
| Sodium Benzoate | 0.25 | 0.25 | 0.25 | 0.25 | 0.25 | 0.25 | 0.25 | 0.25 | 0.25 | 0.25 |
| Ethylene Diamine Tetra Acetic Acid | 0.10 | 0.10 | 0.10 | 0.10 | 0.10 | 0.10 | 0.10 | 0.10 | 0.10 | 0.10 |
| Dimethicone ^{(9, 10, 11)} | | 0.50 ⁽⁹⁾ | | 2.00 ⁽¹⁰⁾ | 1.00 ⁽⁹⁾ | 0.80 ⁽⁹⁾ | | 1.00 ⁽⁹⁾ | 2.00 ⁽⁹⁾ | 0.50 ⁽⁹⁾ |
| Polydecene ⁽¹²⁾ | 0.40 | | | | | | | | | |
| Trimethylolpropane Tricaprylate/Tricaprate ⁽¹³⁾ | 0.10 | | | | | | | | | |
| Cosmetic Pigment (Mica, Titanium Dioxide) ⁽¹⁹⁾ | | 0.10 | | | | | | | | |
| Water and Minors (QS to 100%) | | | | | | | | | | |

**Copolymers**

| EXAMPLE COMPOSITION | **11** | **12** | **13** | **14** | **15** | **16** | **17** | **18** | **19** |
|---|---|---|---|---|---|---|---|---|---|
| Ammonium Laureth Sulfate (AE₃S) | 6.00 | 6.00 | | | 6.00 | 7.50 | 7.50 | | |
| Ammonium Lauryl Sulfate (ALS) | 10.00 | 10.00 | | | 10.00 | 6.50 | 6.50 | 1.00 | |
| Sodium Laureth Sulfate (SE₃S) | | | 6.50 | 6.50 | | | | 6.00 | 6.00 |
| Sodium Lauryl Sulfate (SLS) | | | 5.50 | 5.50 | | | | 7.00 | 10.00 |
| Sodium Lauroamphoacetate ⁽¹⁴⁾ | | | | | | | | | |
| Cocaminopropionic Acrid | | | | 1.00 | | | | | |
| Cocamidopropyl Betaine ⁽¹⁶⁾ | | | | 1.00 | | | | 2.00 | |
| Cocamide MEA | | | 0.80 | 0.80 | | 0.80 | 0.80 | 0.85 | |
| Cetyl Alcohol | | | 0.60 | 0.60 | | 0.60 | 0.60 | | |
| Lauryl Alcohol | | | 0.35 | 0.35 | | | | | |
| Laureth-4 Alcohol | 0.90 | 0.90 | | | 0.90 | | | | 0.90 |
| Dihydrogenated Tallowamidoethyl Hydroxyethylmonium Methosulfate ⁽¹⁷⁾ | | | | | | | | 0.15 | |
| Trimethylammoniopropylmethacrylami de chloride-N-Acrylamide copolymer (20,21) | | 0.50 ⁽²⁰⁾ | 0.10 ⁽²¹⁾ | 1.00 ⁽²⁰⁾ | 0.25 ⁽²¹⁾ | | | 0.05 ⁽²⁰⁾ | |
| Trimethylammoniopropylmethacrylami de chloride-N-vinylpyrrolidone copolymer ^{(22, 23)} | | | | | | 0.40 ⁽²²⁾ | 0.10 ⁽²³⁾ | | |
| Trimethylammoniopropylmethacrylami de chloride-N-Methacrylamidopropyldimethylammoni um methylcarboxylate copolymer ⁽²⁴⁾ | | | | | | | | | 0.40 |
| Trimethylammoniopropylmethacrylami de chloride-N-Behenyl ethoxymethacrylate copolymer ⁽²⁶⁾ | 0.25 | | | | | | | | |
| Ethylene Glycol Distearate | | | 1.50 | 1.50 | | 1.50 | 1.50 | 1.50 | 1.50 |
| Trihydroxystearin ⁽⁷⁾ | 0.10 | 0.10 | | | 0.10 | | | | |
| Polyethylene Glycol (14000) ⁽⁸⁾ | | | 0.17 | 0.17 | | 0.17 | 0.17 | | |
| Fragrance | 0.60 | 0.60 | 0.55 | 0.55 | 0.60 | 0.55 | 0.55 | 0.65 | 0.65 |
| Sodium Chloride | 0.40 | 0.40 | 0.80 | 0.80 | 0.40 | 0.80 | 0.80 | 1.40 | 1.40 |
| Ammonium Xylenesulfonate | | | | | | | | 0.20 | |
| Citric Acid | 0.04 | 0.04 | 0.22 | 0.22 | 0.04 | 0.04 | 0.04 | 0.22 | 0.22 |
| Sodium Citrate | 0.40 | 0.40 | | | 0.40 | 0.40 | 0.40 | | |
| Sodium Benzoate | 0.25 | 0.25 | 0.25 | 0.25 | 0.25 | 0.25 | 0.25 | 0.25 | 0.25 |
| Ethylene Diamine Tetra Acetic Acid | 0.10 | 0.10 | 0.10 | 0.10 | 0.10 | 0.10 | 0.10 | 0.10 | 0.10 |
| Dimethicone ^{(9, 10, 11)} | 1.00 ⁽⁹⁾ | 0.50 ⁽⁹⁾ | 0.50 ⁽⁹⁾ | 2.00 ⁽¹⁰⁾ | 1.00 ⁽⁹⁾ | 0.80 ⁽⁹⁾ | | 1.00 ⁽⁹⁾ | 2.00 ⁽⁹⁾ |
| Polydecene ⁽¹²⁾ | | | | | | | | 0.40 | |
| Trimethylolpropane Tricaprylate/Tricaprate ⁽¹³⁾ | | | | | | | | 0.10 | |
| Cosmetic Pigment (Mica, Titanium Dioxide) ⁽¹⁹⁾ | | 0.10 | | | | | | | |
| Water and Minors (QS to 100%) | | | | | | | | | |

**Comparative Examples**

| EXAMPLE COMPOSITION | **20** | **21** |
|---|---|---|
| Ammonium Laureth Sulfate (AE₃S) | 6.00 | 6.00 |
| Ammonium Lauryl Sulfate (ALS) | 10.00 | 10.00 |
| Laureth-4 Alcohol | 0.90 | 0.90 |
| Trimethylammoniopropylmethacrylami de chloride-N-Acrylamide copolymer (25) | 0.25 | |
| Trihydroxystearin ⁽⁷⁾ | 0.10 | 0.10 |
| Fragrance | 0.60 | 0.60 |
| Sodium Chloride | 0.40 | 0.40 |
| Citric Acid | 0.04 | 0.04 |
| Sodium Citrate | 0.40 | 0.40 |
| Sodium Benzoate | 0.25 | 0.25 |
| Ethylene Diamine Tetra Acetic Acid | 0.10 | 0.10 |
| Dimethicone ^{(9, 10, 11)} | 1.00 ⁽⁹⁾ | 1.00 ⁽⁹⁾ |
| Water and Minors (QS to 100%) | | |

| | | |
|---|---|---|
| (1) HMW MAPTAC (Rhodia) [charge density = 4.5 meq/g, molecular weight ∼ 860,000] (2) HHMW MAPTAC (Rhodia) [charge density = 4.5 meq/g, molecular weight ∼ 1,500,000] (3) Diquat (Rhodia) [charge density = 5.6 meq/g, molecular weight ∼ 252,000] (4) DADMAC (Rhodia) [charge density = 6.2 meq/g, molecular weight ∼ 1,200,000] (5) DADMAC (Rhodia) [charge density = 6.2 meq/g, molecular weight ∼ 175,000] (6) Homopolymer of METAMS (Rhodia) [charge density = 3.5 meq/g, molecular weight ∼ 313,000] (7) Thixcin R (Rheox) (8) PEG 14M (Dow Chemical) (9) Viscasil 330M (Momentive) (10) Dow Corning ® 1664 Emulsion (Dow Corning) (11) Dow Corning ® 2-1865 Microemulsion (Dow Corning) (12) Puresyn 6, MCP-1812 (Mobil) (13) Mobil P43 (Mobil) (14) Miranol Ultra L32 (Rhodia) (15) MACKAM 151C (McIntyre) (16) Tegobetaine F-B (Goldschmidt) (17) Varisoft 110 (Witco) (18) Triqaut (Rhodia) [charge density = 6.07] (19) Timiron MP-149 Diamond Cluster (EMD Chemicals) (20) 1:9 AM:MAPTAC (Rhodia) [charge density = 4.4 meq/g, molecular weight ∼ 1,250,000] (21) 3:7 AM:MAPTAC (Rhodia) [charge density = 4.0 meq/g, molecular weight ∼ 500,000] (22) 1:9 VP:MAPTAC (Rhodia) [charge density = 4.3 meq/g, molecular weight ∼ 242,000] (23) 3:7 VP:MAPTAC (Rhodia) [charge density = 3.7 meq/g, molecular weight ∼503,000] (24) 1:1 AP:MAPTAC (Rhodia) [charge density = 4.0 meq/g, molecular weight ∼243,000] (25) 5:5 AM:MAPTAC (Rhodia) [charge density = 3.4 meq/g, molecular weight ∼ 500,000] (26) 1:9 BEM:MAPTAC (Rhodia) [charge density = 2.6 meq/g] | | |

## Claims

1. Use of a rinse-off shampoo composition for reducing, upon treatment, the surface energy of damaged hair being chemically treated hair, as compared to that measured prior to the treatment, wherein the composition comprises:
i. from 5% to 50% by weight of an anionic surfactant;
ii. from 0.025% to 5% by weight of a synthetic, non-crosslinked, cationic polymer having a cationic charge density of from 4 meq/gm to 7 meq/gm, wherein said synthetic cationic polymer forms lyotropic liquid crystals upon combination with said anionic surfactant; and
iii. water.

2. The use of claim 1, wherein said cationic polymer has an average molecular weight of from 1,000 to 5,000,000, preferably from 10,000 to 2,000,000, more preferably from 100,000 to 2,000,000.

3. The use of claim 1, wherein said cationic polymer comprises monomers selected from the group consisting of dimethylaminoethyl (meth)acrylate, dimethylaminopropyl (meth)acrylate, ditertiobutylaminoethyl (meth)acrylate, dimethylaminomethyl (meth)acrylamide, dimethylaminopropyl (meth)acrylamide; ethylenimine, vinylamine, 2-vinylpyridine, 4-vinylpyridine, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamide chloride, vinylbenzyl trimethyl ammonium chloride, diallyldimethyl ammonium chloride, trimethylammonium ethyl (meth)acrylate chloride, trimethylammonium ethyl (meth)acrylate methyl sulphate, dimethylammonium ethyl (meth)acrylate benzyl chloride, 4-benzoylbenzyl dimethylammonium ethyl acrylate chloride, trimethyl ammonium ethyl (meth)acrylamido chloride, trimethyl ammonium propyl (meth)acrylamido chloride, vinylbenzyl trimethyl ammonium chloride and trimethyl ammonium propyl (meth)acrylamido chloride.

4. The use of claim 1, wherein said cationic polymer comprises monomers selected from the group consisting of diallyldimethyl ammonium chloride.

5. The use of claim 1, wherein said shampoo composition further comprises an ingredient selected from the group consisting of oily conditioning agents, hydrocarbon oils, polyolefins, fatty esters, fluorinated conditioning compounds, fatty alcohols, quaternary ammonium compounds, polyethylene glycols, antidandruff actives, anti-microbial actives, inorganic or synthetic particles, opacifying agents, suspending agents, propellants, paraffinic hydrocarbons, mono or divalent salts, fragrances, vitamins, chelating agents, colorants, pigments, dyes and mixtures thereof.

6. The use of claim 5, wherein said oily conditioning agent is polydimethylsiloxane.

## Patentansprüche

1. Verwendung einer Abspül-Shampoo-Zusammensetzung, um bei der Behandlung die Oberflächenenergie geschädigten Haars, das chemisch behandeltes Haar ist, im Vergleich zu der vor der Behandlung gemessenen Oberflächenenergie zu reduzieren, wobei die Zusammensetzung Folgendes umfasst:
i. von 5 Gew.-% bis 50 Gew.-% ein anionisches Tensid;
ii. von 0,025 Gew.-% bis 5 Gew.-% ein synthetisches, nichtvernetztes, kationisches Polymer mit einer kationischen Ladungsdichte von 4 mÄq/g bis 7 mÄq/g, wobei das synthetische, kationische Polymer bei Kombination mit dem anionischen Tensid lyotrope, flüssige Kristalle bildet; und
iii. Wasser.

2. Verwendung nach Anspruch 1, wobei das kationische Polymer ein durchschnittliches Molekulargewicht von 1.000 bis 5.000.000, vorzugsweise von 10.000 bis 2.000.000, mehr bevorzugt von 100.000 bis 2.000.000 aufweist.

3. Verwendung nach Anspruch 1, wobei das kationische Polymer Monomere umfasst, die ausgewählt sind aus der Gruppe bestehend aus Dimethylaminoethyl-(meth)acrylat, Dimethylaminopropyl(meth)acrylat, Ditertiobutylaminoethyl(meth)acrylat, Dimethylaminomethyl(meth)acrylamid, Dimethylaminopropyl(meth)acrylamid; Ethylenimin, Vinylamin, 2-Vinylpyridin, 4-Vinylpyridin, Trimethylammoniumethyl-(meth)acrylatchlorid, Trimethylammoniumethyl(meth)acrylatmethylsulfat, Dimethylammoniumethyl(meth)acrylatbenzylchlorid, 4-Benzoylbenzyldimethylammoniumethylacrylatchlorid, Trimethylammoniumethyl(meth)acrylamidochlorid, Trimethylammoniumpropyl(meth)acrylamidchlorid, Vinylbenzyltrimethylammoniumchlorid, Diallyldimethylammoniumchlorid, Trimethylammoniumethyl(meth)acrylatchlorid, Trimethylammoniumethyl(meth)acrylatmethylsulfat, Dimethylammoniumethyl(meth)acrylatbenzylchlorid, 4-Benzoylbenzyldimethylammoniumethylacrylatchlorid, Trimethylammoniumethyl-(meth)acrylamidochlorid, Trimethylammoniumpropyl(meth)acrylamidochlorid, Vinylbenzyltrimethylammoniumchlorid und Trimethylammoniumpropyl(meth)acrylamidochlorid.

4. Verwendung nach Anspruch 1, wobei das kationische Polymer Monomere umfasst, die ausgewählt sind aus der Gruppe bestehend aus Diallyldimethylammoniumchlorid.

5. Verwendung nach Anspruch 1, wobei die Shampoo-Zusammensetzung ferner einen Inhaltsstoff umfasst, der ausgewählt ist aus der Gruppe bestehend aus öligen Konditioniermitteln, Kohlenwasserstoffölen, Polyolefinen, Fettestern, fluorierten Konditionierungsverbindungen, Fettalkoholen, quartären Ammoniumverbindungen, Polyethylenglycolen, Antischuppenwirkstoffen, antimikrobiellen Wirkstoffen, anorganischen oder synthetischen Teilchen, Trübungsmitteln, Suspendiermitteln, Treibmitteln, paraffinischen Kohlenwasserstoffen, ein- oder zweiwertigen Salzen, Duftstoffen, Vitaminen, Komplexbildnern, Farbstoffen, Pigmenten, Färbemitteln und Mischungen davon.

6. Verwendung nach Anspruch 5, wobei das ölige Konditioniermittel Polydimethylsiloxan ist.

## Revendications

1. Utilisation d'une composition de shampoing à éliminer par rinçage pour réduire, lors d'un traitement, l'énergie de surface de cheveux endommagés étant des cheveux traités chimiquement, par comparaison avec celle mesurée avant le traitement, dans laquelle la composition comprend :
i. de 5 % à 50 % en poids d'un agent tensioactif anionique ;
ii. de 0,025 % à 5 % en poids d'un polymère cationique synthétique non réticulé ayant une densité de charge cationique allant de 4 méq/gm à 7 méq/gm, dans laquelle ledit polymère cationique synthétique forme des cristaux liquides lyotropiques lors d'une combinaison avec ledit agent tensioactif anionique ; et
iii. de l'eau.

2. Utilisation selon la revendication 1, dans laquelle ledit polymère cationique a une masse moléculaire moyenne allant de 1000 à 5 000 000, de préférence de 10 000 à 2 000 000, plus préférablement de 100 000 à 2 000 000.

3. Utilisation selon la revendication 1, dans laquelle ledit polymère cationique comprend des monomères choisis dans le groupe constitué de (méth)acrylate de diméthylaminoéthyle, (méth)acrylate de diméthylaminopropyle, (méth)acrylate de ditertiobutylaminoéthyle, diméthylaminométhyl-(méth)acrylamide, diméthylaminopropyl-(méth)acrylamide ; éthylène-imine, vinylamine, 2-vinylpyridine, 4-vinylpyridine, chlorure d'éthyl (méth)acrylate de triméthylammonium, méthylsulfate d'éthyl (méth)acrylate de triméthylammonium, chlorure de benzyl-éthyl-(méth)acrylate de diméthylammonium, chlorure d'éthyl-acrylate de diméthylammonium, chlorure de triméthylammonium éthyl-(méth)acrylamido, chlorure de triméthylammonium propyl(méth)acrylamide, chlorure de vinylbenzyl triméthylammonium, chlorure de diallyldiméthylammonium, chlorure d'éthyl (méth)acrylate de triméthylammonium, méthylsulfate d'éthyl (méth)acrylate de triméthylammonium, chlorure de diméthylammonium éthyl(méth)acrylate benzyle, chlorure de 4-benzoylbenzyl diméthylammonium éthyl-acrylate, chlorure de triméthylammonium éthyl(méth)acrylamido, chlorure de triméthylammonium propyl-(méth)acrylamido, chlorure de vinylbenzyl triméthylammonium et chlorure de triméthylammonium propyl (méth)acrylamido.

4. Utilisation selon la revendication 1, dans laquelle ledit polymère cationique comprend des monomères choisis dans le groupe constitué de chlorure de diallyldiméthyl-ammonium.

5. Utilisation selon la revendication 1, dans laquelle ladite composition de shampoing comprend en outre un ingrédient choisi dans le groupe constitué d'agents de conditionnement huileux, huiles hydrocarbures, polyoléfines, esters gras, composés de conditionnement fluorés, alcools gras, composés d'ammonium quaternaire, polyéthylène glycols, principes actifs antipelliculaires, principes actifs antimicrobiens, particules inorganiques ou synthétiques, agents opacifiants, agents de suspension, propulseurs, hydrocarbures paraffiniques, sels mono ou divalents, parfums, vitamines, agents chélatants, colorants, pigments, teintures et leurs mélanges.

6. Utilisation selon la revendication 5, dans laquelle ledit agent de conditionnement huileux est du polydiméthylsiloxane.
